# DEMANDE DE BREVET EUROPEEN

(11) **EP 2 322 231 A1**
(43) Date de publication de la demande: **18.05.2011**
(21) Numéro de dépôt: 10290600.5
(22) Date de dépôt: 09.11.2010
(51) Int. Cl.: A61L 9/20

(54) **Appareils de traitement de l'air interieur par photocatalyse**

(30) Priorité: 17.11.2009 FR 0905500
(71) Demandeur: Photosil Sarl, 75005 Paris (FR)
(72) Inventeur: Bailleux, Christian Charles Léon, 75019 Paris (FR)

(57) **Abrégé**

Appareil de traitement de l'air par photocatalyse pouvant être intégré dans une gaine de climatisation, à forte efficacité bactéricide, faible consommation énergétique, très faible perte de charge, ne nécessitant aucun entretien sur de très longues périodes allant jusqu'à sept ans, réalisé en cumulant l'utilisation d'un photocatalyseur de dioxyde de titane (4) généré *in situ* sur une structure de silice de type feutre ou tissu (5), où le contact avec l'air à traiter se fait à la fois par léchage des faces avant et arrière de la structure photocatalytique, et percolation lors de la traversée de celle-ci, et un système d'irradiation par UV-C au moyen de plusieurs lampes (6), typiquement trois ou quatre, caractérisé en ce que ces lampes sont utilisées successivement, une par une, puis deux par deux, puis toutes ensemble.

## Description

La mise en oeuvre d'innovations en matière de traitement de l'air par photocatalyse a précédemment donné lieu au dépôt de deux brevets : "Support de réaction et réacteur pour le traitement de composés chimiques solides et fluides sous l'action d'un phénomène ondulatoire", et "Nouveaux réacteurs photocatalytiques à base de dioxyde de titane sur support silice pour le traitement de l'air et de l'eau", permettant la réalisation des appareils les plus performants disponibles actuellement.

De nouveaux développements en matière de génie photochimique améliorent ces appareils en réduisant substantiellement leur consommation électrique et leur perte de charge. Ils deviennent ainsi intégrables, sans modification, dans les installations de climatisation existantes, ainsi que dans tous les locaux à usage d'habitation, ou hébergeant des activités sanitaires, éducatives, commerciales, industrielles...

Du fait de ces perfectionnements, la forte efficacité bactéricide de ces appareils, ainsi que leur capacité d'élimination des pollutions d'ordre chimique, en un seul passage, se maintiennent sur de très longues périodes pouvant dépasser huit ans, et cela sans entretien.

De nombreux types de réacteurs photocatalytiques pour le traitement de l'air intérieur ont donné lieu à des brevets, ou à des descriptions dans la littérature technique. Si chacun d'eux a une propriété intéressante sur un point particulier, aucun d'eux ne présente l'ensemble des propriétés suivantes, indispensables à leur développement :
- débit élevé de l'air traité
- très faible perte de charge
- efficacité élevée vis à vis des pollutions chimique et bactérienne en un seul passage,
- faible consommation énergétique
- absence d'entretien
- très longue durée de vie, jusqu'à dix ans et plus.

La réalisation d'appareils dotés de ces propriétés et intégrables, sans modifications dans les systèmes de climatisation existants, a été rendue possible par la combinaison d'éléments ayant déjà donné lieu à brevets :
- support catalyseur à base de silice,
- photocatalyseur TiO₂ synthétisé *in situ* sur le support de silice,
et à de nouveaux développements résultant de mises en oeuvres expérimentales sur site.

### Rappel du principe des réacteurs photocatalytiques

Les radiations lumineuses initient, ou favorisent, certaines réactions chimiques. Les plus actives sont celles dont l'énergie correspond à l'énergie nécessaire à l'activation des liaisons chimiques à mettre en oeuvre. Ces radiations se situent dans le domaine des radiations ultra-violettes (UV).

L'initiation des réactions chimiques telles que la minéralisation des composés organiques volatils (COV) en présence d'air est possible sous irradiation lumineuse (UV-A, B, ou C) si l'on utilise un photocatalyseur.

Parmi les photocatalyseurs les plus actifs figure le Dioxyde de Titane (TiO₂).

L'excitation du TiO₂ revient à créer des paires électron-trou positif qui vont réagir avec les espèces adsorbées, et créer des espèces réactives à partir de l'oxygène de l'air. Les trous positifs et les espèces réactives de l'oxygène provoqueront la dégradation par oxydation des composés organiques tels les Composés Organiques Volatils (COV), jusqu'à leur minéralisation complète (transformation en dioxyde de carbone, eau, azote). Sur ce point on peut se référer à "Photochimie 94" *in* l'Actualité ―Chimique―Supplément au numéro 7 ― décembre 1994.

Les propriétés bactéricides de ces réacteurs sont liées au fait que les microorganismes sont constitués essentiellement de matière organique. La destruction de la matière les constituant, à commencer par leur paroi cellulaire, entraîne leur inertage, puis leur disparition. Cet effet s'ajoute à l'effet classique anti-microbien de l'irradiation ultra-violette.

### Eléments de base du nouveau réacteur photocatalytique

### CHOIX DE LA LONGUEUR D'ONDE D'EXCITATION ULTRA-VIOLETTE.

Le seuil d'excitation du TiO₂ se situe à 0,34 micron. Les lampes UV disponibles pour d'éventuels réacteurs sont les lampes dites "Blue Light" (UV-A), et les lampes UV-C classiques (émission à 0,254 microns).

Les lampes retenues sont les lampes UV-C classiques, car elles ont à la fois :
- la plus longue durée de vie,
- la plus faible consommation énergétique,
- le plus faible coût,
- la plus grande variété de formes et de dimensions.

Toutes ces propriétés sont intéressantes pour les nouveaux réacteurs présentés dans ce brevet, et notamment leur durée de vie à la capacité d'émission nominale de l'ordre de 8 000 heures de fonctionnement ; au-delà, leur capacité d'émission descend de 20% pendant les 4 000 heures suivantes. Leurs constructeurs, soucieux des problèmes écologiques, ont mis au point, ces dernières années, des lampes dites à amalgame, où les traces de mercure, qui déterminent la longueur d'onde d'émission, sont fixées dans un amalgame. Il n'y a, de ce fait, aucune possibilité de libération de mercure libre en cas de bris de ces lampes. Par ailleurs, des accords peuvent être passés avec les fabricants de ces lampes, pour qu'ils reprennent des lampes usagées lors de l'achat de nouvelles lampes.

Le choix final de la lampe, dans la gamme correspondant au modèle géométrique choisi, se fait en se basant sur la détermination en laboratoire de son efficacité : émission UV-C / consommation électrique.

### CHOIX DU SUPPORT SILICE

Pour bénéficier d'une durée de vie importante, le support du photocatalyseur doit être totalement minéral. Un support contenant des parties organiques serait en effet détruit par l'effet photocatalytique lui-même.

Un support totalement transparent aux UV-C assure les propriétés suivantes :
- absence d'absorption des UV-C par le support
- distribution des UV par le support. L'excitation du TiO₂ *via* le support, vient s'ajouter à l'excitation par la surface du TiO₂ irradiée directement.
- absence de certains composés minéraux, tel le sodium, qui réduisent l'effet photocatalytique.

Le matériau constituant le support sera avantageusement à base de verre de silice SiO₂ (commercialisé le plus fréquemment sous le nom de "quartz") de bonne pureté, afin d'être totalement transparent à la longueur d'onde 0,254 micron.

Des verres démixés, dont la partie alcaline a été éliminée par attaque acide, commercialisés sous le nom de "verres lavés", sont cependant utilisables au prix d'une chute de l'efficacité de l'ordre de 20%.

L'intégration des appareils de photocatalyse dans les systèmes de conditionnement d'air peut se faire sans modification des systèmes en place, uniquement à la condition que le débit d'air traversant l'appareil induise seulement une très faible perte de charge, de l'ordre par exemple de 40 pascal, pour un débit de 500 m³/h.

Cette condition peut être réalisée avec des structures de silice de type nappe conciliant dans l'appareil, d'une part la percolation à travers le support revêtu de photocatalyseur, d'autre part le "léchage" de ce même photocatalyseur, en regard des lampes UV. Ainsi, à titre d'exemple, une nappe de 40 dm² de surface - de l'ordre de 80 g/m² - traversée par 500 m³ d'air à l'heure, entraîne seulement une perte de charge de 40 pascal. Des nappes de ce type sont fabriquées et commercialisées jusqu'à des largeurs de un mètre, par plusieurs sociétés, dont en particulier en France par "St Gobain Quartz".

L'ensemble de ces considérations nous a conduit au dépôt d'un premier brevet : "Support de réaction et réacteur pour le traitement de composés chimiques solides et fluides sous l'action d'un phénomène ondulatoire", portant sur l'utilisation de la silice comme support du photocatalyseur avec distribution de la lumière UV vers le photocatalyseur TiO₂ supporté ― N° publication FR 2 765 497.

### LE PHOTOCATALYSEUR TIO₂.

L'intérêt de la synthèse *in situ* avec, simultanément, fixation du photocatalyseur de Dioxyde de Titane (TiO₂) sur le support de silice a donné lieu au dépôt d'un second brevet : "Nouveaux réacteurs photocatalytiques à base de dioxyde de titane sur support silice pour le traitement de l'air et de l'eau". ― N° publication FR 2 806 006. La synthèse *in situ* du TiO₂ sur les nappes de silice est réalisable pour des éléments de dimensions métriques (1 m x 1 m) dans des fours électriques où à gaz de grand volume. Ces dimensions permettent la réalisation de réacteurs photocatalytiques de dimensions variées, et adaptables à tous les systèmes standard, et non-standard, de traitement de l'air, intégrés dans les bâtiments.

### CONCEPTION DE NOUVEAUX REACTEURS PHOTOCATALYTIQUES

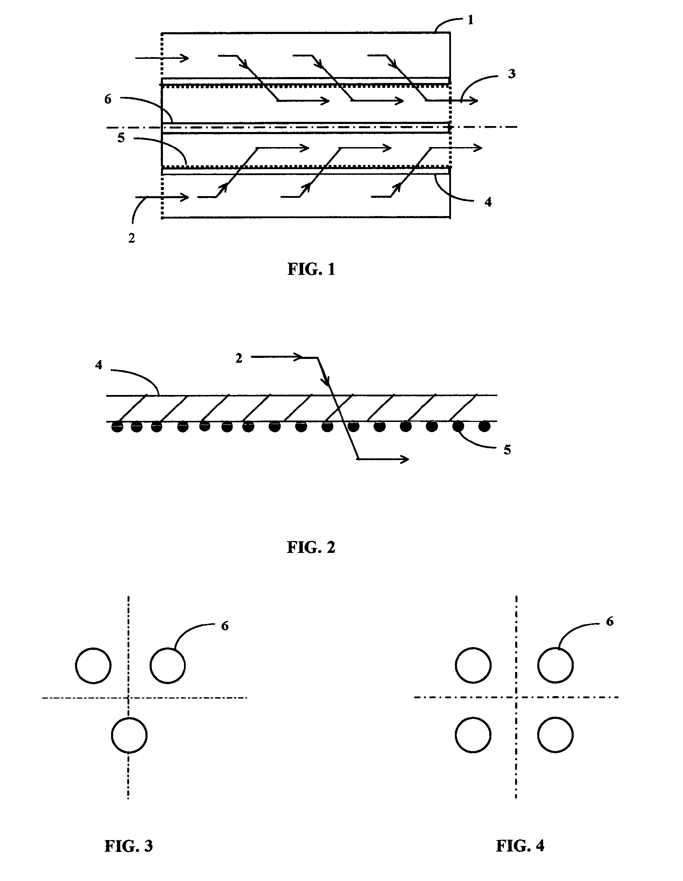

Les éléments constitutifs des nouveaux réacteurs photocatalytiques, présentant simultanément tous les avantages ci-dessus, et dont la **Figure 1** représente schématiquement l'une des multiples variantes possibles, sont : Carter métallique **1** en tôle galvanisée ou en acier inoxydable. Ce carter est doté d'une entrée d'air périphérique **2,** dotée d'un grillage, par où s'effectue l'entrée de l'air à traiter, et d'une sortie axiale **3,** par où s'effectue la sortie de l'air traité, au travers un grillage. Ce carter peut être cylindrique ou parallélépipédique, à axe vertical ou horizontal.

L'air entrant dans l'appareil va passer progressivement au travers du feutre de silice revêtu du photocatalyseur TiO₂ **4,** l'ensemble étant supporté par un grillage métallique d'acier inoxydable à larges mailles **5.** Ce point est présenté de façon détaillé **Figure 2** **.** Ce grillage améliore la tenue du feutre ou tissu de silice revêtu de TiO₂ dans le temps, et sa résistance aux coups de bélier aérauliques. La disponibilité de nappes de silice de dimensions métriques, et la technique de synthèse du photocatalyseur *in situ* sur ces nappes, permettent de répondre, du point de vue dimensionnel, à tous les besoins. Du point de vue de la circulation de l'air à traiter, nous avons donc à la fois un contact avec le photocatalyseur de type léchage, face arrière et face avant, et percolation lors de la traversée de l'ensemble **4.** La combinaison de ces deux types de circulation de l'air vis à vis de l'ensemble support-photocatalyseur permet d'obtenir une perte de charge extrêmement faible au travers de l'appareil, et donc d'introduire l'appareil dans des systèmes existants sans avoir à les modifier.

Au centre de l'appareil se trouvent les lampes UV-C **6.** Dans les systèmes présentés **Figure 3** et **Figure 4****,** les ensembles-lampes comprennent trois et quatre lampes. La disponibilité de lampes UV- C pouvant atteindre un mètre de longueur permet des combinaisons du type de celle représentée **Figure 1****.**

En marche normale, c'est-à-dire hors pic de pollution chimique, une seule lampe fonctionne. La rétrodiffusion de l'émission UV-C captée par le TiO₂ de surface et la distribution par le réseau de fibres de silice assurent une bonne répartition de la lumière UV sur l'ensemble de la matière active TiO₂, même dans ce cas d'émission UV asymétrique par rapport à l'ensemble du photocatalyseur.

En cas de pic de pollution chimique, la mise en marche de toutes les lampes permet de tripler ou de quadrupler la capacité de destruction par minéralisation des polluants de type COV, cela, non seulement en raison de l'augmentation de la puissance d'émission UV, mais aussi de la capacité de distribution des UV vers le photocatalyseur via la silice. Avec un support non transparent aux UV émis, l'augmentation de la puissance d'émission d'un facteur 4, ne se traduirait que par un doublement de la capacité de destruction-minéralisation.

Un tel appareil peut être construit avec une seule lampe, ou deux lampes, mais dans ces deux cas un entretien est à prévoir : remplacement des lampes, en raison de leur durée de vie limitée, et l'on perd la possibilité d'augmenter fortement la capacité de destruction des COV, évoquée ci-dessus dans le cas de pic de pollution.

### Caractéristiques et efficacité d'un appareil réalisé selon les principes décrits ci-dessus.

Des appareils, combinant l'ensemble des paramètres décrits ci-dessus, sont réalisables en adaptant les dimensions, la structure de l'appareil, le nombre de lampes, à chaque cas particulier.

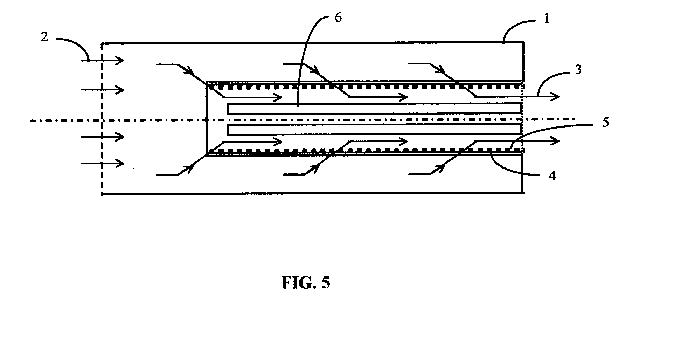

La **Figure 5****,** décrit, à titre d'exemple non limitatif, un appareil réalisé selon les principes présentés ci-dessus. Les composants décrits précédemment s'y retrouvent avec la même indexation. L'enceinte **1** intégrable dans un circuit de climatisation est ici parallèlépipédique, sans que ce soit une obligation, elle a pour dimensions : longueur : 600 mm, largeur : 300 mm, hauteur : 300 mm. Dans l'exemple présenté, l'entrée d'air à traiter s'effectue par la totalité de la face avant de l'appareil, dotée du grillage **2.**

La nappe photocatalytique **4** utilisée a une surface de 40 dm² (4 éléments de 200 mm x 500 mm). Il s'agit d'une nappe de silice de 80g/m², sur laquelle un dépôt de TiO₂ de 25g/m² a été synthétisé selon le procédé breveté indiqué ci-dessus ("Nouveaux réacteurs photocatalytiques à base de dioxyde de titane sur support silice pour le traitement de l'air et de l'eau" ― N° de publication FR 2 806 006).

A l'intérieur prennent place les lampes UV-C. **6,** de puissance électrique unitaire 15 Watt, de 25 mm de diamètre, et de 436 mm de longueur, au nombre de quatre dans cet exemple.

La mesure des caractéristiques aérauliques de l'appareil ainsi réalisé conduit à une perte de charge de 40 Pascal, pour un débit d'air le traversant de 509 m³/h.

La capacité de minéralisation des COV, est de 7 ± 2 g par kwh d'alimentation des lampes. Cette variabilité dépend de la nature chimique du COV minéralisé.

L'utilisation d'une seule lampe permet la minéralisation moyenne de 0,105 g de COV/h.

L'utilisation des quatre lampes ensemble permet la minéralisation moyenne de 0,420 g de COV/h.

Pour un débit de l'air traversant l'appareil de 500 m³/h, on abaisse à chaque passage le niveau de pollution en COV de 160 ppb avec une seule lampe, et de 650 ppb (0,65 ppm) avec les quatre lampes.

L'efficacité bactéricide de l'appareil a été déterminée par impaction selon la norme ISO CD 14698, AFNOR ― NF ― X ― 44 ―110.

Le local retenu pour la caractérisation était une salle de cours universitaire (60 m² - 200 m³). La pollution microbienne de la salle résultait d'une journée entière de cours rassemblant une trentaine d'étudiants. Après leur sortie, les portes ayant été calfeutrées, toutes les mesures ont été réalisées selon la procédure : prélèvement entrée de l'appareil - prélèvement sortie de l'appareil - prélèvement entrée de l'appareil - prélèvement sortie de l'appareil (ensemble systématique de quatre mesures consécutives).

A chaque prélèvement, les boîtes de Pétri, placées dans l'impacteur, piègent l'ensemble des populations microbiennes présentes dans un mètre cube d'air prélevé dans l'atmosphère de la pièce. Les boîtes de Pétri utilisées ont pour référence : AES PCA Standard gélose ― AEB - 520710. Chaque prélèvement était effectué en dix minutes. Le niveau de l'ensemble des populations microbiennes initialement présentes dans la pièce se situait, selon les jours, entre 200 et 300 CFU (Colony Forming Unit).

Les résultats, obtenus avec un appareil rendu autonome, mobile et indépendant de tout système de climatisation par adjonction de ventilateur et d'un filtre de protection à l'entrée, sont les suivants :

| **Caractéristique de l'essai** | **Efficacité mesurée (%)** |
|---|---|
| Feutre 80 g/m² Teneur en TiO₂ : 25 g/m² 4 lampes de 16 W chacune | 94,4 |
| Feutre 80 g/m² Teneur en TiO₂ : 25 g/m² 2 lampes de 16 W chacune | 82,9 |
| Feutre 80 g/m² Teneur en TiO₂ : 25 g/m² 1 lampe de 16 W | 80,9 |

La quantité de TiO₂, fixée sur le feutre de silice, indiquée ci-dessus correspond à l'optimum pour le feutre et la distribution lumineuse de cet appareil. On n'a aucun intérêt à s'éloigner de cet optimum, car on ne ferait que réduire l'efficacité de l'ensemble, même en augmentant la quantité de photocatalyseur. Ainsi, à titre d'exemple, si l'on passe d'une teneur fixée en TiO₂ de 25 g/m², à 50 g/m², l'efficacité mesurée dans le cas de l'appareil à quatre lampes passe de 94,4% à 90,5%.

Cet optimum varie peu avec la géométrie des appareils, car il est essentiellement lié au couple silice-TiO₂,

### Le fonctionnement des appareils "sans entretien".

Les appareils, du type de celui décrit ci-dessus, sont intégrables dans les systèmes standard ou non standard de climatisation d'habitations, d'immeubles, ainsi que dans tous les locaux à usage d'habitation, ou hébergeant des activités sanitaires, éducatives, commerciales, industrielles... L'appareil prend place en aval du filtre du système, ce qui évite l'encrassement de la face arrière de la structure silice-photocatalyseur **4.**

Pour un fonctionnement, hors forte pollution en COV, une seule lampe UV suffit à assurer une bonne efficacité bactéricide (effet supérieur à 80% en un seul passage).

Avec un taux de recirculation de la totalité de l'air du local considéré, de plusieurs fois par heure, on obtient donc, même avec une seule lampe en fonctionnement, une très forte diminution de la pollution microbienne dans le local considéré.

En fonctionnement continu de 24h/24h, la durée de vie des lampes, sans diminution de leur efficacité, est de un an, ce qui permet pour un fonctionnement discontinu de 8h/24h, une durée de vie des lampes de trois ans, sans diminution d'efficacité.

Nous avons donc, pour l'appareil décrit, et en utilisant successivement les lampes une à une, la possibilité d'avoir un fonctionnement continu 24h/24h sur quatre années. Cette durée d'efficacité peut être prolongée par un fonctionnement des lampes 2 à 2, puis les quatre lampes ensemble. Ce qui conduit à une durée de fonctionnement, efficace, sans intervention extérieure sur l'appareil, de : 4 + 2 + 1 = 7 années en fonctionnement 24h/24h.

Pour un fonctionnement hors 24h/24h, soit 8h ou 16h/24h, on dépasse une durée de fonctionnement efficace de 10 années.

Si une forte pollution chimique de type COV survient, on pourra pour une durée déterminée, qui peut être de plusieurs mois, passer à un fonctionnement à quatre lampes. Ce passage pouvant être manuel, ou commandé par un capteur spécifique.

Les systèmes de filtration en place dans tous les systèmes aérauliques intégrés permettent d'éviter l'encrassement du média photocatalytique. De plus, la nappe de silice, revêtue de TiO₂ sur sa face arrière, joue le rôle de filtre complémentaire de celui, ou de ceux, du bâtiment, permettant le maintien d'une bonne irradiation par les lampes de la face en regard.

Dans le cas d'un appareil ne comptant que trois lampes, au lieu de quatre, on obtient une durée de fonctionnement sans intervention de 3 + 1 + 1 = 5 ans en fonctionnement 24h/24h, avec la possibilité, comme précédemment, de passer à tout moment à une utilisation des trois lampes ensemble, en cas de forte pollution chimique de type COV.

## Revendications

**1.** Appareil de traitement de l'air par photocatalyse pouvant être intégré dans une gaine de climatisation, à forte efficacité bactéricide, faible consommation énergétique, très faible perte de charge, ne nécessitant aucun entretien sur de très longues périodes allant jusqu'à sept ans, réalisé en cumulant l'utilisation d'un photocatalyseur de dioxyde de titane **4** généré *in situ* sur une structure de silice de type feutre ou tissu **5,** où le contact avec l'air à traiter se fait à la fois par léchage des faces avant et arrière de la structure photocatalytique, et percolation lors de la traversée de celle-ci, et un système d'irradiation par UV-C au moyen de plusieurs lampes **6,** typiquement trois ou quatre, **caractérisé en ce que** ces lampes sont utilisées successivement, une par une, puis deux par deux, puis toutes ensemble.

**2.** Appareil de traitement de l'air par photocatalyse selon la revendication 1, **caractérisé en ce que**, en cas de forte pollution chimique de type Composés Organiques Volatils, l'augmentation, pour une durée déterminée, de la destruction par minéralisation de ces polluants est obtenue par l'utilisation simultanée de toutes les lampes UV-C de l'appareil.

**3.** Appareil de traitement de l'air par photocatalyse selon l'une des revendications 1 ou 2, où le support catalyseur de silice peut être éventuellement une silice de type "verre lavé".

**4.** Appareil de traitement de l'air par photocatalyse selon l'une des revendications 1, 2 ou 3 où l'appareil est rendu autonome, mobile et indépendant de tout système de climatisation, par adjonction d'un ventilateur et d'un filtre de protection à l'entrée.
